# EUROPÄISCHE PATENTSCHRIFT

(11) **EP 0 683 672 B1**
(45) Veröffentlichungstag und Bekanntmachung des Hinweises auf die Patenterteilung: **12.05.1999**
(21) Anmeldenummer: 94906217.8
(22) Anmeldetag: 03.02.1994
(51) Int. Cl.: A61K 35/78

(54) **EXTRAKT AUS BLÜTEN VON SALVIA OFFICINALIS, VERFAHREN ZU DESSEN HERSTELLUNG UND DESSEN VERWENDUNG**
EXTRACT FROM FLOWERS OF SALVIA OFFICINALIS, PROCESS FOR ITS PREPARATION AND USE THEREOF
EXTRAIT DE FLEURS DE SALVIA OFFICINALIS, SON PROCEDE DE FABRICATION ET SON APPLICATION

(30) Priorität: 10.02.1993 DE 4303823
(43) Veröffentlichungstag der Anmeldung: 29.11.1995
(73) Patentinhaber: HEILMITTELBETRIEB ISERNHAGEN GMBH, D-30916 Isernhagen (DE)
(72) Erfinder: REINHARD, Max, D-61348 Bad Homburg (DE)
(74) Vertreter: Schmidt, Horst, Dr.
(86) Internationale Anmeldenummer: EP9400315
(87) Internationale Veröffentlichungsnummer: WO9417814

(56) Entgegenhaltungen:
- EP-A- 0 147 331
- EP-A- 0 454 097
- WO-A-88/02260

## Beschreibung

Die Erfindung betrifft einen Extrakt aus Blüten von Salvia officinalis (Salbei) sowie ein Verfahren zu dessen Herstellung. Ferner betrifft die Erfindung ein Arzneimittel mit einem Gehalt an dem Extrakt aus Blüten von Salvia officinalis sowie dessen Verwendung bei der Bekämpfung von Durchblutungsstörungen und Bluthochdruck sowie zur Verbesserung der Wundheilung.

EP-A-0 147 331 beschreibt pharmazeutische Zubereitungen enthaltend Extrakte von Ruscus aculeatus in Kombination mit Extrakten von Salvia officinalis zur Verringerung der Permeabilität von kapillaren Blutgefäßen.

Bluthochdruck und damit im Zusammenhang stehende Herz-Kreislauf-Erkrankungen, die nicht selten zum Tode führen, stellen eines der größten medizinischen Probleme dar. Auch Durchblutungsstörungen sind oftmals mit ernsten Komplikationen verbunden. Insbesondere bei älteren Menschen besteht das Problem einer unzureichenden Wundheilung, so daß Wunden in vielen Fällen auch nach langer Zeit nicht verheilen.

Obwohl intensive Anstrengungen unternommen worden sind, um synthetische Arzneimittel oder Naturheilmittel zu entwickeln, die bei den vorstehend genannten Indikationen wirksam sind, besteht nicht zuletzt wegen immer wieder auftretender Nebenwirkungen ein ständiger Bedarf an neuen Arzneimitteln.

Darüber hinaus sind die entwickelten Präparate in der Regel nur für eine der genannten Indikationen einsetzbar. Insbesondere ist bisher kein wirksames Mittel bekannt, das sowohl eine durchblutungsfördernde als auch eine blutdrucksenkende Wirkung zeigt.

Die Verwendung von Pflanzenextrakten als Naturheilmittel ist seit alters her bekannt. Allerdings unterscheiden sich die aus verschiedenen Pflanzenarten, verschiedenen Teilen einer Pflanze oder unter verschiedenen Bedingungen bei der Extraktion gewonnen Extrakte oftmals grundlegend in ihrer Wirkung.

Von der Salbeipflanze ist bisher die Verwendung von wäßrigen Lösungen mit Inhaltsstoffen aus den Blättern gegen Schweißüberproduktion, Katarrhe sowie als Spül- und Gurgelmittel bekannt. Derartige Lösungen werden z.B. durch Behandlung von Salbeiblättern mit heißem Wasser gewonnen. Herkömmliche Verfahren zur Gewinnung von Salbeiextrakten umfassen neben der eigentlichen Extraktionsstufe, z.B. unter Verwendung von Alkohol, eine Stufe zum Abdestillieren des Extraktionsmittels bei Temperaturen über 100°C. Dabei werden viele Inhaltsstoffe thermisch geschädigt oder verändert.

Es ist eine Aufgabe der Erfindung, ein Verfahren bereitzustellen, durch das aus der Salbeipflanze (Salvia officinalis) ein neuartiger Extrakt mit wertvollen Inhaltsstoffen gewonnen werden kann. Dabei soll insbesondere eine thermische Zersetzung der Inhaltsstoffe möglichst weitgehend vermieden werden.

Das Wesen der Erfindung besteht darin, wertvolle Extrakte aus Salbeiblüten zu gewinnen und dabei die Extraktion möglichst schonend durchzuführen, so daß die wertvollen Inhaltsstoffe der Blüte möglichst unverändert in den Extrakt gelangen.

Gegenstand der Erfindung ist ein Verfahren zur Herstellung eines Extraktes aus Salvia officinalis, das dadurch gekennzeichnet ist, daß man Blüten dieser Pflanze extrahiert. Die Extraktion erfolgt vorzugsweise bei einer Temperatur unter 50°C.

Ferner betrifft die Erfindung einen Extrakt aus Blüten von Salvia officinalis, der durch das erfindungsgemäße Verfahren erhalten worden ist.

Außerdem hat die Erfindung ein Arzneimittel zum Gegenstand, das durch einen Gehalt an dem Extrakt aus Blüten von Salvia officinalis gekennzeichnet ist.

Der erfindungsgemäß erhaltene Extrakt eignet sich zur Verwendung bei der Bekämpfung von Durchblutungsstörungen, Bluthochdruck und Wundheilungsstörungen.

Die Extraktion der Blüten von Salvia officinalis kann mit Hilfe von beliebigen Extraktionsmitteln, wie Wasser, organischen Lösungsmitteln oder überkritischem CO₂, erfolgen. Ein Beispiel für ein organisches Lösungsmittel ist Ethanol. Wie bereits erwähnt, soll die Temperatur bei der Extraktion und bei einer sich gegebenenfalls daran anschließenden Stufe zur mindestens teilweisen Entfernung des Extraktionsmittels, z.B. durch Destillation, bei 50°C oder darunter und vorzugsweise bei 40°C oder darunter liegen, um eine thermische Beeinträchtigung der Inhaltsstoffe der Blüte zu verhindern. Dies bedeutet, daß bei der destillativen Trennung, der Druck soweit gesenkt werden muß, daß die genannte Temperaturobergrenze eingehalten werden kann.

Erfindungsgemäß ist die Verwendung von überkritischem CO₂ zur Extraktion von Salbeiblüten besonders bevorzugt, da sie bei niedrigen Temperaturen durchgeführt werden kann und daher besonders schonend ist.

Die Extraktion mit überkritischem CO₂ kann in beliebigen, dafür geeigneten Vorrichtungen erfolgen. Aus den thermodynamischen Eigenschaften von CO₂, nämlich einer kritischen Temperatur von 31,3°C und einem kritischen Druck von 71,5 bar, ergeben sich die unteren Grenzen für die Temperatur und für den Druck bei der Extraktion.

Insbesondere ist es bei der CO₂-Extraktion bevorzugt, bei einer Temperatur von 40°C oder darunter zu arbeiten. Der Druck liegt vorzugsweise im Bereich von 90 bis 300 bar.

Die Extraktion kann solange fortgesetzt werden, bis alle mit überkritischem CO₂ extrahierbaren Inhaltsstoffe aus den Blüten von Salvia officinalis extrahiert worden sind. Dies ist üblicherweise nach einer Dauer des Extraktionsverfahrens von 1 bis 2 Stunden der Fall. Erfindungsgemäß ist es jedoch auch möglich, nur einen Teil der Inhaltsstoffe aus den Blüten von Salvia officinalis zu extrahieren.

Ein Vorteil der Verwendung von überkritischem CO₂ als Extraktionsmittel im Vergleich zur Verwendung anderer Extraktionsmittel, wie Ethanol oder Wasser, besteht darin, daß bei Temperaturen unter 40°C extrahiert werden kann, während z.B. bei einer herkömmlichen alkoholischen Extraktion das Abdestillieren des Ethanols bei Temperaturen von über 100°C erforderlich ist.

Ein weiterer Vorteil der Extraktion mit überkritischem CO₂ besteht darin, daß lösungsmittelfreie Extrakte erhalten werden können. Eine eventuelle ungünstige Beeinflussung der Heilwirkung des Extraktes durch Lösungsmittel, wie Ethanol, kann daher vermieden werden.

Der erfindungsgemäße Extrakt wird vorzugsweise aus Blüten von Salvia officinalis gewonnen. Andere Salbeiarten weisen z.T. erheblich abweichende Inhaltsstoffe auf. Ebenso erhält man bei Extraktion anderer Pflanzenteile von Salvia officinalis, wie Wurzeln, Blätter oder Stengel, eine andere Zusammensetzung des Extraktes.

Die eingesetzten Blüten von Salvia officinalis werden nach der Ernte vorzugsweise getrocknet, und zwar bei einer Temperatur von 40°C oder darunter. Die Trocknungstemperatur wird wie die Extraktionstemperatur vergleichsweise niedrig gewählt, um eine schonende Behandlung der Salbeiblüten zu erlauben. Auch der Einsatz von tiefgefrorenen Blüten kommt in Frage.

Der gemäß dem erfindungsgemäßen Verfahren gewonnene Extrakt aus Blüten von Salvia officinalis wird in pastenartiger Konsistenz erhalten. Dieser Extrakt kann als wirksamer Bestandteil eines Arzneimittels verwendet werden.

Das erfindungsgemäße Arzneimittel kann in einer beliebigen Darreichungsform hergestellt werden. Dazu wird der Extrakt aus Blüten von Salvia officinalis mit beliebigen geeigneten Verdünnungsmitteln, Füllstoffen oder dergl. versetzt und anschließend in die gewünschte Darreichungsform überführt. Es bestehen keine speziellen Beschränkungen hinsichtlich des Verhältnisses von Extrakt zu Verdünnungsmittel, Füllstoff oder dergl. Die Herstellung des Arzneimittels kann nach üblicher pharmazeutischer Praxis erfolgen.

Zur innerlichen Anwendung kann das erfindungsgemäße Arzneimittel oral, z.B. in Form einer Tablette, einer Pastille, einer Kapsel, eines Pulvers, eines Elixiers, eines Sirups oder dergl., oder parenteral als Injektionspräparat verabreicht werden. Zur Herstellung einer Tablette wird der durch CO₂-Extraktion gewonnene Extrakt mit einem üblichen Tablettenfüllstoff, wie Lactose, vermischt. Das Gemisch wird dann zu Tabletten verpreßt.

Das erfindungsgemäße Arzneimittel kann auch äußerlich, z.B. in Form einer Salbe, einer Creme oder einer Tinktur, angewandt werden. Zur Herstellung einer Salbe wird der CO₂-Extrakt mit einer Salbengrundlage, wie einer fetten Salbengrundlage, vermischt. Ein Beispiel für eine fette Salbengrundlage ist Vaseline.

Das erfindungsgemäße Arzneimittel zeigt eine blutdruckregulierende und insbesondere eine blutdrucksenkende Wirkung. Dementsprechend kann es z.B. in Form von Tabletten zum Zweck der Bekämpfung von Bluthochdruck verwendet werden.

Das erfindungsgemäße Arzneimittel kann auch angewandt werden, um die Durchblutung zu fördern. Dementsprechend kann es z.B. in Form einer Salbe zum Zweck der Bekämpfung von Durchblutungsstörungen verwendet werden. Ferner kann mit dem erfindungsgemäßen Arzneimittel auch die Wundheilung verbessert werden. Dadurch ist z.B. die Heilung von Wunden bei alten Menschen möglich, die mit anderen Mitteln nicht zu heilen sind. Dementsprechend kann das erfindungsgemäße Arzneimittel zur Bekämpfung einer unzureichenden Wundheilung verwendet werden.

Nachstehend wird die Erfindung durch Beispiele näher erläutert.

### Beispiel 1

In diesem Beispiel wird die Herstellung eines CO₂-Gesamtextraktes beschrieben. Dazu wurden handgepflückte, getrocknete Blüten von Salvia officinalis 2 Stunden bei einem Druck von 300 bar (vollständige Extraktion) und einer Temperatur von 40°C mit CO₂ extrahiert. Aus 15,2 kg Salbeiblüten wurden dabei 623 g Extrakt gewonnen. Dies entspricht einer Extraktausbeute von 4,1 %. Der Extrakt fiel als Paste an.

### Beispiel 2

In diesem Beispiel wird die Gewinnung eines CO₂-Selektivextraktes beschrieben. Die Extraktionsbedingungen umfaßten eine Extraktionsszeit von 2 Stunden, einen Druck von 90 bar (selektive Extraktion) und eine Temperatur von 40°C. Aus 1,7 kg eingesetzten getrockneten Blüten von Salvia officinalis wurden dabei 14 g Extrakt gewonnen. Dies entspricht einer Extraktausbeute von 0,8 %. Der Extrakt fiel als Paste an.

### Beispiel 3

Die gemäß den Beispielen 1 und 2 erhaltenen pastenförmigen Extrakte wurden mit pulverförmiger Lactose vermischt. Die Gemische wurden mit einer üblichen Tablettenpresse zu Tabletten verpreßt. Die Tabletten enthielten 4 bis 5 mg Extrakt und wiesen einen Durchmesser von 9 mm und eine Dicke von 3 mm auf.

### Beispiel 4

Der Extrakt, der nach dem in Beispiel 1 oder 2 beschriebenen Verfahren erhalten wurde, wurde mit Vaseline als Salbengrundlage gemischt. Die Salbe enthielt 0,1 % Extrakt, bezogen auf die Salbengrundlage.

### Beispiel 5

In diesem Beispiel wird die blutdrucksenkende Wirkung von Tabletten mit einem Gehalt an dem erfindungsgemäßen Extrakt beschrieben.

Bei Einnahme einer Tablette (vgl. Beispiel 3) täglich sank der Blutdruck bei einer männlichen Person im Alter von 35 Jahren von 145 zu 102 nach 4 Tagen auf 123 zu 95. Bei einer anderen männlichen Person im Alter von 35 Jahren sank der Blutdruck von 147 zu 105 nach 6 Tagen auf 121 zu 88 bei einer täglichen Dosis von einer Tablette.

## Patentansprüche

1. Verwendung eines Extraktes aus Blüten von Salvia officinalis zur Herstellung eines Arzneimittels für die Bekämpfung von Durchblutungsstörungen, Bluthochdruck und Wundheilungsstörungen.

2. Verfahren zur Herstellung eines Extraktes aus *Salvia officinalis*, dadurch gekennzeichnet, daß man die Blüten dieser Pflanze bei einer Temperatur von 50°C oder darunter mit überkritischem CO₂ bei einem Druck im Bereich von 90 bis 300 bar extrahiert.

3. Verfahren nach Anspruch 2, dadurch gekennzeichnet, daß die Extraktion bei einer Temperatur von 40°C oder darunter erfolgt.

4. Verfahren nach einem der Ansprüche 2 bis 3, dadurch gekennzeichnet, daß die Blüten von *Salvia officinalis* vor der Extraktion zunächst bei einer Temperatur von 40°C oder darunter getrocknet werden.

5. Verfahren nach einem der Ansprüche 3 bis 4, dadurch gekennzeichnet, daß eine im wesentlichen vollständige Extraktion vorgenommen wird.

6. Verfahren nach einem der Ansprüche 3 bis 4, dadurch gekennzeichnet, daß eine nur teilweise Extraktion vorgenommen wird.

7. Extrakt aus Blüten von *Salvia officinalis*, dadurch gekennzeichnet, daß er durch ein Verfahren nach einem der Ansprüche 2 bis 6 erhalten worden ist.

8. Arzneimittel, gekennzeichnet durch einen Gehalt an Extrakt aus Blüten von *Salvia officinalis* nach Anspruch 7.

## Claims

1. Use of an extract from the flowers of Salvia officinalis for producing a medicament for combating circulatory disorders, high blood pressure and wound healing disorders.

2. A method of producing an extract of Salvia officinalis, wherein the flowers of said plant are extracted with supercritical CO₂ at a temperature of 50°C or below at a pressure in the range from 90 to 300 bar.

3. The method as set forth in claim 2, wherein wherein the extraction is performed at a temperature of 40°C or below.

4. The method as set forth in any of the claims 2 to 3, wherein the flowers of Salvia officinalis prior to the extraction are dried at a temperature of 40°C or below.

5. The method as set forth in any of the claims 3 to 4, wherein an essentially complete extraction is performed.

6. The method as set forth in any of the claims 3 to 4, wherein an extraction only in part is performed.

7. An extract from the flowers of Salvia officinalis, in which said extract is obtained by a method according to any of the claims 2 to 6.

8. A medicament, including a content of extract from flowers of Salvia officinalis according to claim 7.

## Revendications

1. Utilisation d'un extrait de fleurs de Salvia officinalis pour produire un médicament pour lutter contre les troubles de la circulation sanguine, l'hypertension et les problèmes de cicatrisation.

2. Procédé de production d'un extrait de Salvia officinalis, caractérisé en ce qu'on réalise l'extraction des fleurs de cette plante à une température de 50 °C ou moins avec du CO₂ surcritique sous une pression comprise entre 90 et 300 bar.

3. Procédé selon la revendication 2, caractérisé en ce que l'extraction est effectuée à une température de 40 °C ou moins.

4. Procédé selon l'une des revendications 2 ou 3, caractérisé en ce que les fleurs de Salvia officinalis sont séchées avant l'extraction à une température de 40 °C ou moins.

5. Procédé selon l'une des revendication 3 ou 4, caractérisé en ce qu'on réalise une extraction pratiquement complète.

6. Procédé selon l'une des revendication 3 ou 4, caractérisé en ce qu'on ne réalise qu'une extraction partielle.

7. Extrait de fleurs de Salvia officinalis, caractérisé en ce qu'il est obtenu par un procédé selon l'une quelconque des revendications 2 à 6.

8. Médicament caractérisé en ce qu'il contient de l'extrait de fleurs de Salvia officinalis selon la revendication 7.
